# EUROPÄISCHE PATENTSCHRIFT

(11) **EP 2 573 005 B1**
(45) Veröffentlichungstag und Bekanntmachung des Hinweises auf die Patenterteilung: **01.11.2017**
(21) Anmeldenummer: 12183511.0
(22) Anmeldetag: 07.09.2012
(51) Int. Cl.: B65D 47/18, B65D 47/20, A61F 9/00, B01L 3/00

(54) **Tropfenspender**
Drop dispenser
Distributeur compte-gouttes

(30) Priorität: 23.09.2011 DE 102011083355
(43) Veröffentlichungstag der Anmeldung: 27.03.2013
(73) Patentinhaber: Aptar Radolfzell GmbH, 78315 Radolfzell (DE)
(72) Erfinder: Wochele, Matthias, 78239 Rielasingen (DE)
(74) Vertreter: Patentanwaltskanzlei Cartagena

(56) Entgegenhaltungen:
- EP-A1- 0 401 022
- EP-A1- 0 431 885
- WO-A1-84/00707
- WO-A1-2006/043295
- WO-A2-2008/068775

## Beschreibung

Die Erfindung betrifft einen Tropfenspender zur Abgabe einer pharmazeutischen Flüssigkeit in Tropfenform. Dabei weist ein solcher gattungsgemäßer Tropfenspender ein Gehäuse und einen Flüssigkeitsspeicher sowie eine Austragöffnung zur Ausbringung der Flüssigkeit auf, die eine Wandung des Gehäuses durchdringt und der ein Auslassventil zugeordnet ist.

Tropfenspender sind zur Verabreichung verschiedener Medikamente aus dem Stand der Technik, wie z.B. WO2006043295, allgemein bekannt. Sie verfügen über einen Flüssigkeitsspeicher, der beispielsweise als Quetschflasche ausgebildet sein kann und der durch Zusammendrücken bei gleichzeitig nach unten weisender Austragöffnung eine Förderung der Flüssigkeit durch die Austragöffnung bewirkt. Außenseitig der Austragöffnung bildet sich bei einem solchen Tropfenspender bestimmungsgemäß ein Tropfen, der sich vom Spender trennt, wenn er eine ausreichende Größe aufweist, die zu einer Abschnürung des überwiegenden Teils der ausgetragenen Flüssigkeitsmenge in Form eines Haupttropfens führt. In der Umgebung der Austragöffnung verbleibt üblicherweise ein technisch kaum vermeidbarer Resttropfen.

Da die Dosierung von Medikamenten, die in Tropfenform ausgebracht werden, üblicherweise über die Anzahl der Tropfen erfolgt, ist die Erzielung einer immer gleichbleibenden Größe des sich abtrennenden Haupttropfens von großer Bedeutung.

Aus dem Dokument EP 0 431 885 A1 ist eine Tropfer-Spitze für einen Tropferspender bekannt, welche einen im Wesentlichen kreiszylindrischen Grundkörper aufweist, in welchem ein sich in Richtung eines freien Endes aufweitender Kanal vorgesehen ist. Die Tropfer-Spitze weist an dem freien Ende eine Kugelform auf, durch welche eine Abgabe von Tropfen einheitlicher Größe unabhängig von einem Neigungswinkel der Tropfer-Spitze erzielt werden soll.

Als problematisch hat sich in der Vergangenheit der oben genannte Resttropfen herausgestellt. Bei Tropfenspendern, die nicht über ein der Austragöffnung zugeordnetes Auslassventil verfügen, wird dieser Resttropfen üblicherweise durch den sich im Flüssigkeitsspeicher zuvor gebildeten Unterdruck wieder in die Flasche eingesogen. Bei gattungsgemäßen Tropfenspendern, die ein üblicherweise druckabhängig öffnendes Auslassventil aufweisen, ist der Rückweg in den Tropfenspender hinein für den Resttropfen jedoch nach Ende des Austragvorgangs verschlossen, so dass der Resttropfen außenseitig der Austragöffnung verbleibt. Dies ist problematisch, da dieser Resttropfen Träger einer Kontamination werden kann. Es ist daher wünschenswert, dass der Resttropfen so schnell als möglich verdunstet.

### Aufgabe und Lösung

Aufgabe der Erfindung ist es, einen gattungsgemäßen Tropfenspender dahingehend weiterzubilden, dass dieser ein besonders schnelles Verdunsten des Resttropfens gestattet. Aufgabe ist es weiterhin, einen Tropfenspender zur Verfügung zu stellen, bei dem das Flüssigkeitsvolumen eines jeden Tropfens weitgehend konstant ist.

Erfindungsgemäß wird diese Aufgabe gemäß einem ersten Aspekt der Erfindung durch einen Tropfenspender gemäß Anspruch 1 erfüllt. Die Austragöffnung ist von einer Tropfenbildungsfläche auf der Außenseite des Gehäuses umgeben, wobei diese Tropfenbildungsfläche zumindest in einem Außenbereich - bezogen auf eine Ausrichtung des Tropfenspenders mit vertikal nach unten weisender Austragöffnung - eine sich nach unten und eine zur Austragöffnung verjüngende Formgebung aufweist und darüber hinaus hydrophil ausgebildet ist.

Die Erfindung betrifft somit insbesondere die Formgebung und Ausgestaltung der Tropfenbildungsfläche, welches jene Fläche ist, an der bestimmungsgemäß der abzugebende Tropfen haftet, bevor er sich zumindest zum überwiegenden Teil vom Spender trennt. Unter der sich nach unten verjüngenden Formgebung des Außenbereichs dieser Tropfenbildungsfläche ist insbesondere eine kegelartige oder kegelstumpfartige Formgebung zu verstehen, wobei auch z.B. ballige Formen möglich sind. Die Mantelfläche dessen ist außenseitig der Austragöffnung angeordnet und hydrophil ausgebildet.

Unter einer hydrophilen Ausbildung dieser genannten Fläche wird verstanden, dass unter Referenzbedingungen von Wasser bei 20°C ein das Maß der Hydrophilie wiedergebender Kontaktwinkel zwischen dem genannten Wasser und der Fläche 20° oder weniger beträgt, insbesondere vorzugsweise 10° oder weniger.

Die Kombination der sich verjüngenden und hydrophil ausgebildeten Tropfenbildungsfläche, die die Austragöffnung umgibt, hat sich insbesondere im Hinblick auf die Verdunstungszeit des Resttropfens als vorteilhaft herausgestellt. Durch diese Gestaltung wird verglichen mit einer ebenen Gestaltung einer Tropfenbildungsfläche und unter der Annahme eines gleichen Außendurchmessers des am Spender verbleibenden Resttropfens eine größere Fläche bereitgestellt, auf die sich dieser Resttropfen verteilt. Zudem ist beobachtet worden, dass der Resttropfen üblicherweise ein geringeres Volumen aufweist.

Diese vergrößerte Fläche, auf die sich der volumetrisch verkleinerte Resttropfen verteilt, führt zu einer Verkürzung der Trockenzeit. So konnte unter im Übrigen gleichbleibenden Bedingungen beobachtet werden, dass ein Wassertropfen an einer ebenen Tropfenbildungsfläche verglichen mit einer erfindungsgemäß zumindest abschnittsweise konisch/verjüngend und hydrophil ausgebildeten Tropfenbildungsfläche gleichen Außendurchmessers eine etwa 4-fache Verdunstungszeit aufweist.

Grundsätzlich ist dieser Vorteil der verringerten Verdunstungszeit auch gegeben, wenn nicht nur eine begrenzte Tropfenbildungsfläche, sondern stattdessen der gesamte Spender an seiner Außenseite hydrophil ausgebildet ist. Um eine definierte Tropfengröße zu erzeugen, wird es jedoch als vorteilhaft angesehen, wenn die Tropfenbildungsfläche örtlich begrenzt ist. Dies kann beispielsweise dadurch erzielt werden, dass die Tropfenbildungsfläche von einer Umgebungsfläche umgeben ist, die eine geringere Hydrophilie als die Tropfenbildungsfläche aufweist. Vorzugsweise ist die Hydrophilie in der die Tropfenbildungsfläche umgebenden Umgebungsfläche dergestalt, dass sie unter den oben genannten Randbedingungen zu einem Kontaktwinkel größer 20°, vorzugsweise größer 45° führt. Der somit vorgesehene Sprung hinsichtlich der Hydrophilie zwischen der Tropfenbildungsfläche und der Umgebungsfläche bewirkt, dass die Abtrennung des Haupttropfens vom Resttropfen üblicherweise stattfindet, ohne dass Flüssigkeit zuvor über die Außengrenze der Tropfenbildungsfläche hinweg in den Bereich der Umgebungsfläche gelangt.

Alternativ oder zusätzlich ist es möglich, die Tropfenbildungsfläche außenseitig durch eine Grenzkante zu begrenzen, die einen Krümmungsradius von maximal 0,2 mm aufweist. Eine solche Grenzkante führt ebenfalls zu einer Verstärkung der Neigung des Haupttropfens, sich vom Resttropfen zu lösen, noch bevor Flüssigkeit über die genannte Grenzkante hinweg die hydrophile Tropfenbildungsfläche verlässt. Vorzugsweise wird die Grenzkante zusammen mit der Umgebungsfläche geringerer Hydrophilie realisiert. Denkbar ist jedoch durchaus auch eine Gestaltung, bei der die Fläche außerhalb der Grenzkante ebenfalls hydrophil ausgebildet ist und die Grenzkante somit alleine Verantwortung dafür trägt, dass bei der Tropfenbildung die Flüssigkeit die Grenzen der Tropfenbildungsfläche nicht überschreitet.

Die Tropfenbildungsfläche ist derart bemessen, dass eine Parallelprojektion der Tropfenbildungsfläche in einer Horizontalebene - bezogen auf eine Ausrichtung des Tropfenspenders mit vertikal nach unten weisender Austragöffnung - eine Projektionsfläche zwischen 0,4 mm² und 24 mm² aufweist, vorzugsweise eine Projektionsfläche zwischen 3 mm² und 7 mm².

Dabei ist die durch die Parallelprojektion auf einer Horizontalebene gebildete Projektionsfläche als die alleine durch die Außenkanten der Tropfenbildungsfläche bestimmte Projektionsfläche zu begreifen. Die Projektion der Austragöffnung ist somit ebenfalls Teil dieser Projektionsfläche. Das genannte Größenintervall zwischen 0,4 mm² und 24 mm² hat sich für die Bildung von Tropfen zwischen 10 µl und 80 µl als geeignet herausgestellt. Die häufig gewünschte Tropfengröße von etwa 30 bis 40 µl wird durch das kleinere der genannten Intervalle erzielt.

Die Tropfenbildungsfläche sowie die Außenflächen im Bereich der Austragöffnung summieren sich aufgrund der sich verjüngenden oder konischen Formgebung naturgemäß auf eine Gesamtfläche, die größer als die genannten Werte ist, da diese sich auf die Projektion beziehen. Gerade diese gegenüber der Projektionsfläche vergrößerte tatsächliche Tropfenbildungsfläche gewährleistet die verkürzte Verdunstungszeit.

Hinsichtlich der Erzeugung der gewünschten Hydrophilie an der Tropfenbildungsfläche sind mehrere Möglichkeiten gegeben. So ist bei einer ersten bevorzugten Variante vorgesehen, dass eine partielle Beschichtung eines die Wandung des Gehäuses bildenden Kunststoffs genutzt wird, um die Hydrophilie herzustellen. Sofern eine Umgebungsfläche geringerer Hydrophilie um die Tropfenbildungsfläche herum vorgesehen ist, so kann diese Umgebungsfläche durch einen unbeschichteten Abschnitt dieses Kunststoffs gebildet sein. Auch kann die Hydrophilie durch partielles Bestrahlen erzielt werden. Bei dieser Gestaltung erfolgt somit eine Beschichtung / Bestrahlung eines zuvor nicht hydrophilen Kunststoffs zur Erzeugung der Hydrophilie. Die Grundeigenschaften des Kunststoffs können im Bereich der Umgebungsfläche zur dort vorgesehenen schwächeren Hydrophilie genutzt werden.

Alternativ zu dieser Gestaltung ist es möglich, dass die Hydrophilie der Tropfenbildungsfläche durch einen Gehäuseabschnitt hergestellt wird, der als Ganzes aus einem hydrophilen Material besteht. Sofern in diesem Falle eine Umgebungsfläche geringerer Hydrophilie gewünscht ist, so kann diese insbesondere dadurch hergestellt sein, dass die Oberfläche eines zweiten Gehäuseabschnitts diese Umgebungsfläche bildet, der mit dem ersten Gehäuseabschnitt fest verbunden ist. Insbesondere kann der erste Gehäuseabschnitt in einer Ausnehmung des zweiten Gehäuseabschnitts eingesetzt sein. Somit können bei dieser Variante Beschichtungsvorgänge oder Bestrahlungsvorgänge von einzelnen Gehäuseabschnitten entfallen. Stattdessen werden Gehäuseabschnitte aus Materialien mit unterschiedlicher Hydrophilie genutzt.

Zur Hydrophilisierung der Oberflächen kann diese beispielsweise mit einem Polymer mit hydrophilen Eigenschaften beschichtet werden. Beispiele für Polymere mit hydrophilen Eigenschaften sind 2-Hydroxy-Äthyl-Methacrylat (HEMA), Glyzerin-Methacrylat (GMA), Glyzerin-MethylMethacrylat (GMMA), N-Vinylpyrrolidon (NVP), Methacrylsäure (MAA), Methyl-Metacrylat (MMA), Polyurethan (PU), Polyvinylalkohol (PVA) oder Polydextrose. Diese Materialien eigenen sich nicht nur als Beschichtungsmaterialien. Stattdessen kann das entsprechende Bauteil auch als ganzes aus ihnen bestehen. Auch das Aufdampfen eines Metalls wie Aluminium bewirkt in der Regel einen deutlichen Anstieg der Hydrophilie.

Auch kann der fragliche Oberflächenbereich beispielsweise auch durch eine Plasmabehandlung hydrophilisiert werden. Diese wird in der Regel in einem Plasmareaktor bei sehr niedrigem Druck durchgeführt. Das Plasma enthält reaktive Spezies wie z.B. Ionen und freie Radikale, die mit der Oberfläche reagieren und diese chemisch modifizieren können, stets in Abhängigkeit von der Natur den physikalischen Eigenschaften des Plasmas. Für Hydrophilisierungen wird häufig ein Sauerstoff- oder ein Argonplasma eingesetzt.

Ergänzend wird in Hinblick auf die Herstellung der gewünschten Hydrophilie auch auf die DE 112006001297 T5 und die DE 102007049587 A1 verwiesen, deren Offenbarung hinsichtlich der Herstellung hydrophiler Materialien und Oberflächen durch Verweis zum Gegenstand der vorliegenden Offenbarung gemacht wird.

Als besonders vorteilhaft hat sich die Verwendung nano-vernetzter siliciumorganischer Verbindungen herausgestellt.

Gemäß einem zweiten Aspekt der Erfindung, der vorzugsweise zusammen mit dem bereits genannten ersten Aspekt der Erfindung realisiert ist, ist bei einem gattungsgemäßen Tropfenspender vorgesehen, dass die Tropfenbildungsfläche durch eine Außenoberfläche eines Tropfenbildungskörpers des Gehäuses gebildet wird, wobei dieser Tropfenbildungskörper die Form einer Kugelkalotte aufweist, deren Kugeloberfläche größer ist als die Kugeloberfläche einer Halbkugel gleichen Durchmessers.

Bei einer solchen Gestaltung ist somit ein Tropfenbildungskörper vorgesehen, der wie ein einer Halbkugel übersteigender Abschnitt einer Kugel anmutet. Die zwangsläufig gegebene Abweichung von der Kugelform im Bereich der Auslassöffnung ist hierbei unbeachtlich. Es kommt hinsichtlich dieses Aspektes der Erfindung lediglich darauf an, dass die Tropfenbildungsfläche einen Äquatorialbereich des kugelartigen Körpers bildet und diesen zu beiden Seiten hin überschreitet. Der Tropfenbildungskörper ist mit anderweitigen Abschnitten des Gehäuses vorzugsweise durch einen gegenüber dem Durchmesser der Kugel verjüngten Steg verbunden. Die Ausgestaltung des Tropfenbildungskörpers als ein eine Halbkugel größenmäßig übersteigender Körper ermöglicht es, gleichbleibende Tropfengrößen unabhängig von einer genau vertikalen Ausrichtung des Tropfenspenders zu erzielen. So ist es zumindest erreichbar, dass ein Anwinkeln des Tropfenspenders gegenüber einer Vertikalausrichtung um bis zu 15° keinerlei Auswirkungen auf die Größe des Tropfens hat.

### Kurzbeschreibung der Zeichnungen

Weitere Aspekte und Vorteile der Erfindung ergeben sich außer aus den Ansprüchen auch aus der nachfolgenden Beschreibung bevorzugter Ausführungsbeispiele der Erfindung, die anhand der Figuren erläutert werden. Dabei zeigen:
- Fig. 1: einen erfindungsgemäßen Tropfenspender in einer Gesamtdarstellung,
- Fig. 2a bis 2c: eine erste Ausgestaltung der Auslasseinheit des Tropfenspenders der Fig. 1,
- Fig. 3: eine zweite alternative Ausgestaltung der Auslasseinheit des Tropfenspenders und
- Fig. 4a bis 4c: eine dritte alternative Ausgestaltung der Auslasseinheit des Tropfenspenders.

### Detaillierte Beschreibung der Ausführungsbeispiele

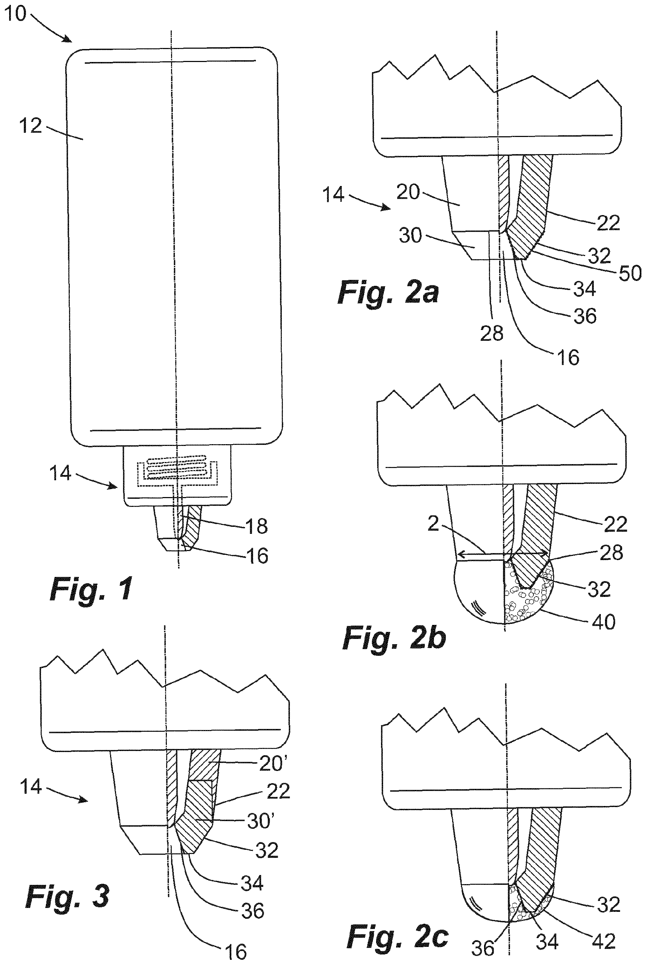

Fig. 1 zeigt einen erfindungsgemäßen Tropfenspender in einer Gesamtdarstellung. Der Tropfenspender 10 verfügt über einen Flüssigkeitsspeicher 12, der als Quetschflasche ausgebildet ist. In einer Nutzposition ist eine Auslasseinheit 14 des Tropfenspenders 10 nach unten gerichtet. Diese Auslasseinheit verfügt über eine Auslassöffnung 16, welche im verschlossenen Zustand des Spenders 10 durch einen Ventilstift verschlossen ist. Der Ventilstift ist Teil eines in Fig. 1 gestrichelt angedeuteten Ventils 18, welches in Abhängigkeit des Flüssigkeitsdrucks innerhalb des Tropfenspenders 10 öffnet und schließt. Zum Austrag von Flüssigkeit aus dem Flüssigkeitsspeicher 12 durch die Austrittsöffnung 16 hindurch wird der Flüssigkeitsspeicher 12 manuell zusammengedrückt. Dies bewirkt eine Druckerhöhung und ein Öffnen des Ventils 18 durch Verlagerung des Ventilstifts. Die austretende Flüssigkeit sammelt sich bestimmungsgemäß im bereich der Austrittsöffnung 16 und bildet dort einen Tropfen, der nach Erreichen einer bestimmten Größe sich zum überwiegenden Teil vom Spender löst, während ein kleinerer Teil, Resttropfen genannt, im Bereich der Außenfläche der Auslasseinheit 14 verbleibt.

Die Fig. 2a bis 2c zeigen die Auslasseinheit 14 des Spenders der Fig. 1 in vergrößerter Form. Unter Bezugnahme auf Fig. 2a wird die bauliche Gestaltung der Auslasseinheit 14 näher erläutert. Es ist zu erkennen, dass die Außenform der Auslasseinheit 14 zwei aneinander angrenzende kegelstumpfförmige Abschnitte 20, 30 aufweist. Die Mantelfläche 32 des kegelförmigen Abschnitts 30 dient bestimmungsgemäß der Anhaftung eines abzugebenden Tropfens, bevor dieser sich vom Tropfenspender 10 löst. Ebenfalls der Anhaftung dieses Tropfens dienen die Stirnfläche 34 sowie die Innenfläche 36, die die Austrittsöffnung 16 umgeben. Um diese Anhaftung zu erleichtern und insbesondere um zu bewirken, dass die an der Austrittsöffnung 16 austretende Flüssigkeit entlang der Mantelfläche 32 gegen die Schwerkraft nach oben wandert, sind die genannten Flächen 32, 34, 36 hydrophil ausgestaltet. In Fig. 2a, 2b und 2c wird dies durch die hier eingezeichnete gestrichelte Linie verdeutlicht. In diesem Bereich der gestrichelten Linie ist die Oberfläche des einstückigen Körpers, der sowohl den Kegelabschnitt 30 als auch den Kegelabschnitt 20 bildet, durch eine Beschichtung 50 oder aber alternativ durch Bestrahlung hydrophil ausgebildet. Die an die Mantelfläche 32 angrenzende Mantelfläche 22 des oberen kegelstumpfförmigen Abschnitts 20 ist dagegen nicht hydrophil ausgebildet, sondern weist eine unveränderte Materialoberfläche auf.

Bei dem Material des die Kegelstumpfabschnitte 20, 30 bildenden Bauteils kann es sich beispielsweise um HDPE handeln. Dieses ist als Material nicht hydrophil, so dass ein Kontaktwinkel mit Wasser als Flüssigkeit in etwa 80° beträgt. Im Bereich der hydrophilen Oberflächen 32, 34, 36 beträgt aufgrund der Beschichtung dieser Kontaktwinkel weniger als 5°.

Zwischen den Mantelflächen 22, 32 ist eine scharfkantige Grenzkante 28 vorgesehen, die einen Krümmungsradius von etwa 0,2 mm aufweist. Diese Grenzkante unterstützt die nachfolgend erläuterte Funktionsweise beim Austragen eines Tropfens.

Fig. 2b zeigt den Vorgang der Tropfenerzeugung. Durch eine Kraftbeaufschlagung des Flüssigkeitsspeichers 12 wird die Flüssigkeit bei leicht geöffnetem Auslassventil durch die Austragöffnung 16 hindurchgedrückt und sammelt sich in der in Fig. 2b skizzierten Weise. Dabei ist besonders hervorzuheben, dass die Flüssigkeit außenseitig der Mantelfläche 32 an dieser entlang nach oben kriecht, die Grenzkante 28 aufgrund von deren geometrischer Gestaltung und aufgrund der hydrophoben Ausbildung der Mantelfläche 22 jedoch nicht überschreitet. Somit ist der Außendurchmesser 2 der Tropfenbildungsfläche klar definiert.

Mit ansteigendem Volumen des Tropfens 40 steigt dessen Neigung zur Ablösung vom Tropfenspender 10. Bei Erreichen einer insbesondere durch die Geometrie der Tropfenbildungsfläche bedingten Größe des Tropfens wird dieser in nicht dargestellter Art und Weise eingeschnürt, so dass sich der überwiegende Teil des Tropfens, der Haupttropfen, vom Tropfenspender 10 löst. Am Tropfenspender 10 verbleibt lediglich noch der Resttropfen 42, der in Fig. 2c dargestellt ist. Das Zurückbleiben eines Resttropfens ist bislang nicht vollständig vermeidbar. Wie Fig. 2c zeigt, ist durch beschriebene und dargestellte Gestaltung jedoch eine sehr vorteilhafte Ausprägung dieses Resttropfens 42 zu bewirken. Aufgrund der recht großen Tropfenbildungsfläche 32, 34, 36, die erheblich größer als eine Ebene Kreisfläche mit dem Durchmesser 2 ist, verteilt sich die verbleibende Flüssigkeitsmenge recht gut, so dass die Schichtdicke der Flüssigkeit gering ist. Der Resttropfen 42 gemäß Fig. 2c wird daher schnell verdunsten. Neben der vergleichsweise großen Tropfenbildungsfläche 32, 34, 36 ist hierfür auch verantwortlich, dass gleichsam ein wesentlicher Teil des Innenvolumens des Resttropfens 42 durch den Kegelabschnitt 30 ausgefüllt ist.

Fig. 3 zeigt eine alternative Gestaltung, die hinsichtlich ihrer Funktionsweise mit der vorangegangenen Gestaltung übereinstimmt. Die Besonderheit bei der Gestaltung gemäß Fig. 3 liegt darin, dass die Mantelfläche 32, die Stirnfläche 34 und die zur Auslassöffnung 16 hin weisende Fläche 36 an einem einen separaten Gehäuseabschnitt bildenden separaten Körper 30' vorgesehen sind, welcher aus einem in sich hydrophilen Material besteht. Dieses Material kann beispielsweise durch eingelagerte Zusätze hydrophil gemacht worden sein. Dieser separate Körper 30' ist in einer Ausnehmung eines die Mantelfläche 22 bildenden Gehäuseabschnitts 20' eingesetzt und dort beispielsweise durch eine Presspassung gehalten.

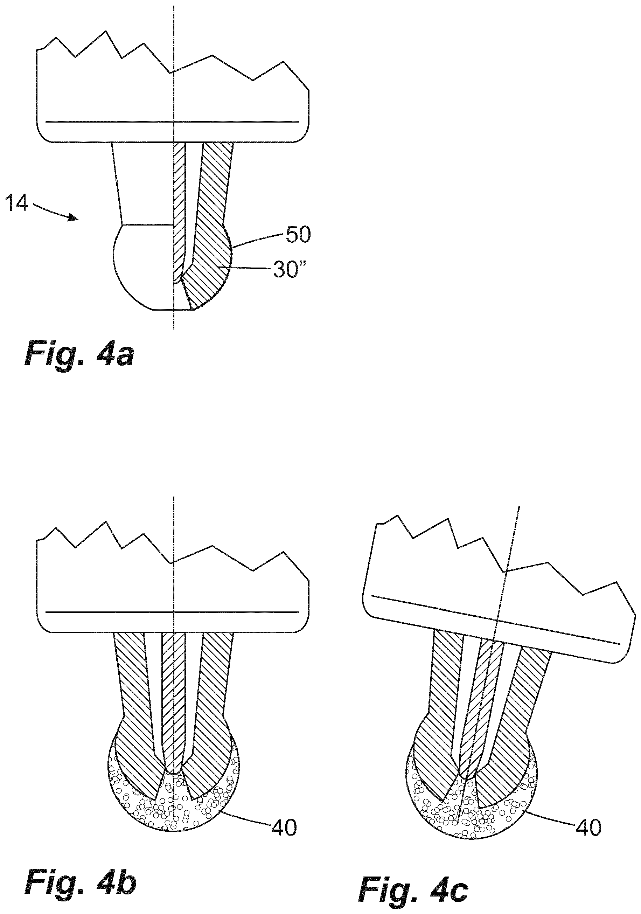

Die Ausgestaltung der Fig. 4a bis 4c ähneln wiederum der Ausgestaltung der Fig. 2a bis 2c, da auch hier ein einstückiger die Außenseite der Auslasseinheit 14 bildender Körper vorgesehen ist, der lediglich partiell mit einer Beschichtung 50 versehen ist, die zu hydrophilen Eigenschaften führt. Abweichend von der Gestaltung der Fig. 2a bis 2c ist hier allerdings die Besonderheit verwirklicht, dass die Außenfläche der Auslasseinheit 14 abschnittsförmig die Form einer Kugelkalotte 30" aufweist, deren Außenseite in der genannten Art mit der Beschichtung 50 versehen ist. Die Wirkung dieser Außenbeschichtung und der genannten Formgebung ähnelt der im Hinblick auf die Fig. 2a bis 2c geschilderten Wirkung. Wiederum kann durch die vergrößerte Oberfläche erreicht werden, dass ein Resttropfen, der nach dem Austrag verbleibt, schnell verdunsten kann.

Die Besonderheit der kugelkalottenförmigen Oberfläche ergibt sich dabei insbesondere aus den Fig. 4b und 4c. Es ist zu erkennen, dass die Kugelkalottenform dazu führt, dass das Volumen des sich bildenden Tropfens 40 und die Größe des Tropfens 40 im Augenblick des Ablösens vom Tropfenspender 10 nicht mehr von der präzisen Ausrichtung des Tropfenspenders abhängen. So zeigt Fig. 4c, dass auch bei einem Verkippen des Tropfenspenders 10 um in etwa 15° das Volumen des Tropfens nicht beeinflusst wird. Somit wird auch bei nicht ganz präziser Handhabung des Tropfenspenders 10 eine genaue Dosierung gewährleistet.

## Patentansprüche

1. Tropfenspender (10) zur Abgabe einer pharmazeutischen Flüssigkeit in Tropfenform (40) mit
- einem Gehäuse,
- einem Flüssigkeitsspeicher (12) und
- einer Austragöffnung (16) zur Ausbringung der Flüssigkeit, die eine Wandung des Gehäuses durchdringt und der ein Auslassventil (18) zugeordnet ist,
**wobei**
- die Austragöffnung (16) von einer Tropfenbildungsfläche (32, 34, 36) auf der Außenseite des Gehäuses umgeben ist,
- diese Tropfenbildungsfläche (32, 34, 36) zumindest in einem Außenbereich (36), bezogen auf eine Ausrichtung des Tropfenspenders mit vertikal nach unten weisender Austragöffnung (16), eine sich nach unten und zur Austragöffnung (16) verjüngende Formgebung aufweist **dadurch gekennzeichnet, dass**
- diese Tropfenbildungsfläche (32, 34, 36) hydrophil ausgebildet ist, wobei ein das Maß der Hydrophilie wiedergebender Kontaktwinkel zwischen Wasser unter Referenzbedingungen bei 20°C und der Tropfenbildungsfläche (32, 34, 36) 20° oder weniger beträgt.

2. Tropfenspender (10) nach Anspruch 1,
**dadurch gekennzeichnet, dass**
die Tropfenbildungsfläche (32, 34, 36) von einer Umgebungsfläche (22) umgeben ist, die eine geringere Hydrophilie als die Tropfenbildungsfläche (32, 34, 36) aufweist.

3. Tropfenspender (10) nach Anspruch 1 oder 2,
**dadurch gekennzeichnet, dass**
die Tropfenbildungsfläche (32, 34, 36) außenseitig durch eine Grenzkante (28) begrenzt ist, wobei diese Grenzkante (28) einen Krümmungsradius von maximal 0,2 mm aufweist.

4. Tropfenspender (10) nach einem der vorstehenden Ansprüche,
**dadurch gekennzeichnet, dass**
die Tropfenbildungsfläche (32, 34, 36) derart bemessen ist, dass eine Parallelprojektion der Tropfenbildungsfläche (32, 34, 36) in eine Horizontalebene - bezogen auf eine Ausrichtung des Tropfenspenders mit vertikal nach unten weisender Austragöffnungeine Projektionsfläche zwischen 0,4 mm² und 24 mm² aufweist, vorzugsweise eine Projektionsfläche zwischen 3 mm² und 7 mm².

5. Tropfenspender (10) nach einem der vorstehenden Ansprüche,
**dadurch gekennzeichnet, dass**
die Hydrophilie der Tropfenbildungsfläche (32, 34, 36) durch partielle Beschichtung eines die Wandung des Gehäuses bildenden Kunststoffs hergestellt ist, wobei vorzugsweise die Umgebungsfläche (22) durch einen unbeschichteten Abschnitt dieses Kunststoffs gebildet ist.

6. Tropfenspender (10) nach einem der Ansprüche 1 bis 4,
**dadurch gekennzeichnet, dass**
die Hydrophilie der Tropfenbildungsfläche (32, 34, 36) durch einen Gehäuseabschnitt (30') hergestellt ist, der als ganzes aus einem hydrophilen Material besteht, wobei vorzugsweise die Umgebungsfläche (22) durch die Oberfläche eines zweiten Gehäuseabschnitts (20') gebildet ist, in den der erste Gehäuseabschnitt (30') eingesetzt ist.

7. Tropfenspender (10) nach einem der vorstehenden Ansprüche oder nach dem Oberbegriff von Anspruch 1,
**dadurch gekennzeichnet, dass**
die Tropfenbildungsfläche durch eine Außenoberfläche eines Tropfenbildungskörpers (30") des Gehäuses gebildet wird, wobei dieser Tropfenbildungskörper die Form einer Kugelkalotte aufweist, deren Kugeloberfläche die Kugeloberfläche einer Halbkugel gleichen Durchmessers übersteigt.

## Claims

1. Drop dispenser (10) for releasing a pharmaceutical liquid in drop form (40), with
- a housing,
- a liquid reservoir (12) and
- a discharge opening (16) for discharging the liquid, which discharge opening (16) penetrates a wall of the housing and is assigned an outlet valve (18),
wherein
- the discharge opening (16) is surrounded by a drop-forming surface (32, 34, 36) on the outside of the housing,
- this drop-forming surface (32, 34, 36), at least in an outer area (36) with respect to an orientation of the drop dispenser with the discharge opening (16) pointing vertically downwards, has a shape tapering downwards and towards the discharge opening (16),
**characterized in that**
- this drop-forming surface (32, 34, 36) is hydrophilic, wherein a contact angle, giving the degree of hydrophilicity, between water under reference conditions at 20°C and the drop-forming surface (32, 34, 36) is 20° or less.

2. Drop dispenser (10) according to Claim 1, **characterized in that** the drop-forming surface (32, 34, 36) is surrounded by a surrounding surface (22) which has a lower hydrophilicity than the drop-forming surface (32, 34, 36).

3. Drop dispenser (10) according to Claim 1 or 2, **characterized in that** the drop-forming surface (32, 34, 36) is delimited on the outside by a boundary edge (28), wherein this boundary edge (28) has a maximum radius of curvature of 0.2 mm.

4. Drop dispenser (10) according to one of the preceding claims, **characterized in that** the drop-forming surface (32, 34, 36) is dimensioned in such a way that a parallel projection of the drop-forming surface (32, 34, 36) in a horizontal plane, with respect to an orientation of the drop dispenser with the discharge opening pointing vertically downwards, has a projection area of between 0.4 mm² and 24 mm², preferably a projection area of between 3 mm² and 7 mm².

5. Drop dispenser (10) according to one of the preceding claims, **characterized in that** the hydrophilicity of the drop-forming surface (32, 34, 36) is produced by partial coating of a plastic that forms the wall of the housing, wherein the surrounding surface (22) is preferably formed by an uncoated section of this plastic.

6. Drop dispenser (10) according to one of Claims 1 to 4, **characterized in that** the hydrophilicity of the drop-forming surface (32, 34, 36) is produced by a housing section (30') which as a whole is composed of a hydrophilic material, wherein the surrounding surface (22) is preferably formed by the surface of a second housing section (20') into which the first housing section (30') is inserted.

7. Drop dispenser (10) according to one of the preceding claims or according to the preamble of Claim 1, **characterized in that** the drop-forming surface is formed by an outer face of a drop-forming body (30") of the housing, wherein this drop-forming body has the shape of a spherical cap, the sphere surface of which exceeds the sphere surface of a hemisphere of the same diameter.

## Revendications

1. Distributeur (10) de gouttes pour distribuer un liquide pharmaceutique sous forme de gouttes (40) présentant
- un boîtier,
- un réservoir (12) à liquide et
- une ouverture de sortie (16) pour faire sortir le liquide, qui pénètre dans une paroi du boîtier et à laquelle est associée une soupape de sortie (18),
- l'ouverture de sortie (16) étant entourée d'une surface (32, 34, 36) de formation de gouttes sur le côté externe du boîtier,
- cette surface (32, 34, 36) présentant une forme se rétrécissant vers le bas et vers l'ouverture de sortie (16) au moins dans une zone externe (36), par rapport à une orientation du distributeur de gouttes, l'ouverture de sortie (16) étant dirigée verticalement vers le bas, **caractérisé en ce que**
- cette surface (32, 34, 36) de formation de gouttes est réalisée de manière hydrophile, un angle de contact représentant le degré d'hydrophilie entre l'eau dans des conditions de référence à 20°C et la surface (32, 34, 36) de formation de gouttes étant de 20° ou moins.

2. Distributeur (10) de gouttes selon la revendication 1, **caractérisé en ce que** la surface (32, 34, 36) de formation de gouttes est entourée par une surface environnante (22) qui présente une hydrophilie inférieure à celle de la surface (32, 34, 36) de formation de gouttes.

3. Distributeur (10) de gouttes selon la revendication 1 ou 2, **caractérisé en ce que** la surface (32, 34, 36) de formation de gouttes est délimitée, côté externe, par un bord de délimitation (28), ce bord de délimitation (28) présentant un rayon de courbure d'au maximum 0,2 mm.

4. Distributeur (10) de gouttes selon l'une quelconque des revendications précédentes, **caractérisé en ce que** la surface (32, 34, 36) de formation de gouttes est dimensionnée de manière telle qu'une projection parallèle de la surface (32, 34, 36) de formation de gouttes présente, dans un plan horizontal - par rapport à une orientation du distributeur de gouttes, l'ouverture de sortie étant dirigée verticalement vers le bas - une surface de projection entre 0,4 mm² et 24 mm², de préférence une surface de projection entre 3mm² et 7 mm².

5. Distributeur (10) de gouttes selon l'une quelconque des revendications précédentes, **caractérisé en ce que** l'hydrophilie de la surface (32, 34, 36) de formation de gouttes est réalisée par un revêtement partiel d'un matériau synthétique formant la paroi du boîtier, la surface environnante (22) étant de préférence formée par une section non revêtue de ce matériau synthétique.

6. Distributeur (10) de gouttes selon l'une quelconque des revendications 1 à 4, **caractérisé en ce que** l'hydrophilie de la surface (32, 34, 36) de formation de gouttes est formée par une section (30') de boîtier, qui est constituée dans son ensemble par un matériau hydrophile, la surface environnante (22) étant de préférence formée par la surface d'une deuxième section (20') de boîtier dans laquelle la première section (30') de boîtier a été placée.

7. Distributeur (10) de gouttes selon l'une quelconque des revendications précédentes ou selon le préambule de la revendication 1, **caractérisé en ce que** la surface de formation de gouttes est formée par une surface externe d'un corps (30") de formation de gouttes du boîtier, ce corps de formation de gouttes présentant la forme d'une calotte sphérique dont la surface sphérique dépasse de la surface sphérique d'une demi-sphère de même diamètre.
